# EUROPÄISCHE PATENTSCHRIFT

(11) **EP 1 867 728 B1**
(45) Veröffentlichungstag und Bekanntmachung des Hinweises auf die Patenterteilung: **14.01.2009**
(21) Anmeldenummer: 07450110.7
(22) Anmeldetag: 15.06.2007
(51) Int. Cl.: C12P 13/02, C12P 17/12, C12P 17/16, C12P 17/18, C12P 19/42, C12P 25/00, A01H 3/04

(54) **Verfahren zur Herstellung einer mit B Vitaminen angereicherten Pflanze**
Process for the preparation of a plant enriched with B vitamins
Procédé de préparation d'une plante enrichie en vitamines du groupe B

(30) Priorität: 16.06.2006 AT 10292006
(43) Veröffentlichungstag der Anmeldung: 19.12.2007
(73) Patentinhaber: JHS-Privatstiftung, 1020 Wien (AT)
(72) Erfinder: Fuchs, Norbert, 5571 Mariapfarr (AT); Loidl, Rupert, 5571 Mariapfarr (AT); Sadeghi, Behzad, 1100 Wien (AT)
(74) Vertreter: Sonn & Partner Patentanwälte

(56) Entgegenhaltungen:
- EP-A- 1 371 283
- MOZAFAR, A. & OERTLI, J.J.: "Uptake and Transport of Thiamin (Vitamin B1) by Barley and Soybean" JOURNAL OF PLANT PHYSIOLOGY, Bd. 4, Nr. 139, 1992, Seiten 436-442, XP001051110

## Beschreibung

Die vorliegende Erfindung betrifft die Anreicherung und Modifizierung von Vitaminen in pflanzlichen Nahrungsmitteln.

Vitamine sind organische Verbindungen, die für eine Vielzahl biochemischer Funktionen in kleinen Mengen benötigt werden. Der menschliche Organismus kann Vitamine nicht oder nicht in ausreichender Menge ausbilden und muss sie deshalb über die Nahrung zuführen. Als Co-Faktoren oder prosthetische Gruppen von zellulären Enzymen üben biologisch aktive Vitamine vor allem katalytische Funktionen aus: sie ermöglichen als BioKatalysatoren Stoffwechselreaktionen unter physiologischen Bedingungen, die ohne ihren katalytischen Einfluss nur unter hohem Druck oder hohen Temperaturen möglich wären. Während Mensch und Tier nicht oder nur eingeschränkt in der Lage sind, Vitamine endogen zu produzieren, sind Pflanzen und "niedere" Organismen (z.B. Bakterien und/oder Algen) in der Lage, aus geeigneten Kohlenstoff-, Stickstoff-, Mineral- und natürlichen Energiequellen (Sonnenlicht) diese Biokatalysatoren auf biochemischem Wege aufzubauen. Der Mensch ist somit auf die regelmäßige und ausreichende Zufuhr von Vitaminen angewiesen, um einen reibungslosen Energie-, Bau- und Funktionsstoffwechsel seines Organismus aufrecht zu erhalten. Sämtliche Wachstums-, Abwehr- und Regenerationsprozesse des menschlichen Organismus sind somit auf ausreichende Körperbestände von Vitaminen angewiesen.

Anhand von Vitaminpräparaten wird versucht, direkt die Vitamingehalte in Lebensmitteln zu erhöhen. Dazu wird beispielsweise in einem Lebensmittel durch Beimischen der Vitaminpräparate der Vitamin-Spiegel komplementiert. Pflanzen oder pflanzliche Produkte können zu diesem Zweck mit Vitaminpräparaten besprüht werden.

Einen alternativen Weg zur Erhöhung des Vitamingehalts wird in der US 2004/0115288 A1 beschrieben. Durch eine kontrollierte Prozedur von Pflanzenschnitten, abgewechselt mit definierten Wachstumsphasen wird der Vitamin B-Gehalt in Pflanzen auf natürliche Weise erhöht.

Eine Methode um generell den Nährstoffgehalt in Pflanzen zu erhöhen wird in der US 5,973,224 offenbart, wobei Pflanzen-Embryos in ausgewählten Elektrolyt-Lösungen inkubiert werden.

Die GB 500 284, GB 485 097 und GB 484 981 betreffen die Behandlung von Pflanzensamen mit wachstumsfördernden Stoffen, darunter Vitamin B1, Laktoflavin (Vit. B2), und Biotin (Vit. B7) .

Vitaminbehandlungen haben zugleich auch den Vorteil, dass bei Pflanzen das Wachstum beschleunigt und somit der Ertrag gesteigert werden kann. So wird in der EP 524 411 A1 vorgeschlagen, Pflanzensamen in einer Vitamin B1 (auch Thiamin oder Aneurin bezeichnet)-Zusammensetzung mit ggf. einem Fungizid behandelt.

Die EP 1 371 283 A2 beschreibt das Einweichen von Pflanzensamen in einer Vitamin B12-Lösung, um in den Pflanzen den Vitamin B12-Gehalt zu erhöhen. Vitamin B12 wird von Pflanzen nicht selbst produziert, sondern ebenfalls nur aus der Erde (wo es von Bakterien produziert wird) aufgenommen. Als Vitamin B12 werden gemäß dieser Schrift sämtliche Formen von Vitamin B12, nämlich Cobalamin, Cyanocobalamin, Hydroxycobalamin, Methylcobalamin und Adenosylcobalamin bezeichnet. Der Vitamin B12-Bedarf eines Menschen ist relativ gering, nämlich 1 µg, aber ebenso gering ist auch der Gehalt in Pflanzen. Durch die Pflanzensamen-Behandlung kann dieses Vitamin in Pflanzen auf bis zu 0,5 µg/g angereichert werden.

Das Dokument Mozafar, A. & Oertli, J.J.: "Uptake and Transport of Thiamin (Vitamin B1) by Barley and Soybean" (JOURNAL OF PLANT PHYSIOLOGY, Bd. 4, Nr. 139, 436-442, 1992) offenbart eine Untersuchung zur Aufnahme und zur Verteilung von Vitamin B in Pflanzen, wobei die Bätter der Keimlinge während des Keimungsprozesses mit einer Lösung des B Vitamins, die 0,01 % Tween-20 enthält, benetzt werden.

Die WO 2000/013502 A1 betrifft ein Düngemittel zur Aufbringung auf Blättern durch z.B. Besprühen.

Die US 2004/0063582 A1 betrifft Saatmaterial mit anhaftenden Mikronährstoffen, Vitaminen und Pestiziden.

Die WO 1999/026470 A1 betrifft ein Nährmedium für Pflanzen, in dem Pflanzensamen großgezogen werden können.

Die GB 1 108 164 beschreibt eine Nährstofflösung, in der Samen für ein schnelleres Wachstum gekeimt werden können.

Die WO 2005/063002 A1 betrifft die künstliche somatische Embryogenese in Baumwollpflanzen in einer Nährstofflösung.

Die Erkenntnis um die elementare Notwendigkeit einer regelmäßigen und ausreichenden Vitaminzufuhr für Tier und Mensch hat in den letzten Jahrzehnten zur chemischen, biochemischen und fermentativen Entwicklung und Herstellung isolierter Vitamine im industriellen Maßstab geführt. Hat sich die Versorgung von Tier und Mensch mit dieser Art hergestellter Vitamine überwiegend als notwendig und sinnvoll erwiesen, haben - insbesondere während der letzten 10 Jahre - einige groß angelegte Interventionsstudien gezeigt, dass die Zufuhr einzelner, isolierter und hoch dosierter Vitamine unter bestimmten Umständen und bei einigen Bevölkerungsgruppen auch negative Effekte zeigen können:

Zwei groß angelegte skandinavische Studien zeigten, dass die Zufuhr isolierten Beta-Carotins die Lungenkrebs-Sterblichkeitsrate bei Rauchern und Asbest-Arbeitern erhöht (ATBC-Studie sowie CARET-Studie).

Eine weitere Doppelblind-Studie an 4.000 Diabetikern bzw. cardiovaskulär erkrankten Personen zeigte, dass die Zufuhr isolierten Vitamin E's das Risiko für Herzkomplikationen in der Verum-Gruppe erhöhte (HOPE-Studie).

Eine kürzlich präsentierte norwegische Studie mit 3.800 Herzinfarkt-Patienten zeigte, dass die regelmäßige Einnahme einer Kombination aus synthetischem Vitamin B6, Vitamin B12 sowie Folsäure das Herzinfarkt- oder Schlaganfall-Risiko in der Verum-Gruppe im Vergleich zur Placebo-Gruppe um 20% erhöhte (NORVIT-Studie).

Es ist daher ein Ziel der vorliegenden Erfindung, Vitamine in ausreichender Menge zur Verfügung zu stellen, die auch für den Konsumenten verträglich oder verträglicher sind, ebenso wie biologisch aktive Vitamin-B-Komplexe in organisch gebundener Form mit hoher Bioverfügbarkeit zu erhalten.

Dies wird durch die Bereitstellung von Pflanzen mit hohen Vitamingehalten bewerkstelligt, die in ihrem Pflanzensamenstadium mit einer Vitaminlösung getränkt wurden. Erfindungsgegenstand ist ein Verfahren zur Produktion von organisch gebundenem Vitamin B, vorzugsweise ausgewählt aus Vitamin B1, B2, B3, B5, B6, B7, B9, B12 oder Mischungen hievon, in Pflanzen, wobei keimfähige Pflanzensamen in einer Lösung des jeweiligen Vitamins bzw. der Vitaminmischung anfangs getränkt und während des Keimungsprozesses mit den jeweiligen Vitamin-Lösungen besprüht werden. Unter Keimung versteht man das beginnende Wachstum nach der ruhenden Phase des Pflanzensamens. Dabei wächst der Keimling aus dem Samen heraus. Anders werden die keimfähigen Pflanzensamen getränkt und eingeweicht (angekeimt) und anschließend durch Besprühen zur Keimung gebracht. Die Keimung kann als abgeschlossen angesehen werden, wenn der Pflanzensamenkörper aufgebraucht und ggf. die unverwertbare Hülle des Samens (wenn vorhanden) abgeworfen wurde. Durch anschließendes Besprühen wird der Keimling zu Vitamin B-angereicherten Pflanzenkeimlingen kultiviert. Vorzugsweise ist das Vitamin ausgewählt aus Vitamin B3, B5, B6 oder B9. Die anfängliche Tränkung (Einweichen) kann durchgeführt werden bis ein Anschwellen beobachtbar ist. Nach dem Einweichen wird der Keimling kultiviert und mit der Vitaminlösung besprüht, sodass ein die Keimung und ein Wachstum erwirkt wird. Die kann 1x oder mehrmals am Tag vorgenommen werden. Besprüht wird bis die Keimung ersichtlich ist, z.B. je nach Pflanzenart 1 bis 20 Tage, insbesondere mehr als 1, 2, 3, 4, 5, 6, 7 oder 8 Tage und gegebenenfalls weniger als 30, 25, 20, 18, 15, 12, 10, 8, 7, 5 oder 3 Tage. Nach dem Besprühen oder dem Beginn der Keimung kann in einem Zeitrahmen von bis zu oder mindestens 20, 22, 24, 26, 28, 30, 32, 34, 36, 38, 40, 42, 44, 45, 46, 48, 50, 52, 54, 56, 58, 60, 62, 64, 66, 68, 70, 75, 80, 85, 90, 95, 100, 105, 110, 115, 120, 130, 140, 150 oder 160 Stunden besprüht werden. Da die Pflanzen für einen relativ kurzen Zeitrahmen behandelt werden, können sie in bevorzugten Ausführungsformen frei von Pflanzenschutzmittel wie z.B. Pestiziden und Fungiziden, gehalten werden.

Im Gegensatz zu den exemplarisch angeführten Studien zu Vitaminpräparaten, die potenziell negative Effekte durch langzeitige Zufuhr synthetischer Einzel-Vitamine oder Vitamin-Kombinationen zeigen, gibt es weltweit keine Studie, die negative Effekte durch die regelmäßige Zufuhr natürlicher, biologisch aktiver pflanzlicher Vitamine aufzeigt.

Ein Grund für die Diskrepanz in der biologischen Wirkung synthetischer und natürlich gewachsener Vitamine mag darin liegen, dass natürliche Vitamine in der pflanzlichen Natur meist gebunden und komplex vorliegen, während synthetische Vitamine chemisch ungebundene, isolierte und klar definierte Molekülverbindungen darstellen.

So ist das (synthetische) Aneurin (als freie Base oder als Salz) in essbaren Pflanzen in dieser Form nicht existent, sondern kann nur als biologisch aktives Thiaminpyrophosphat oder Thiamintriphosphat isoliert werden.

Vitamin B2 ist synthetisch als Riboflavin definiert, während es im pflanzlichen Organismus vorwiegend in einer Unzahl so genannter Flavoproteine, als FADH oder z.B. auch als FMN in seiner biologisch aktiven Form vorkommt.

Vitamin B3 wiederum stellt sich, chemisch hergestellt, als Nikotinsäure (Niacin) oder Nikotinsäureamid dar, während die pflanzliche, biologisch aktive Form von Vitamin B3 vorwiegend als NADH und NADPH (in reduzierter Form) bzw. als NAD und NADP (in oxidierter Form) in Erscheinung tritt.

Synthetische Pantothensäure (Vit. B5) wiederum kommt in freier Form im pflanzlichen Organismus praktisch überhaupt nicht vor, sondern entfaltet dort seine biologische Funktion als biologisch aktives Coenzym A.

Vitamin B6 wiederum kann im Labor als Pyridoxol, Pyridoxal oder Pyridoxamin synthetisiert werden, während die (pflanzliche) biologische aktive Form vorwiegend als Pyridoxalphosphat auftritt.

Synthetisches Biotin (Vit. B7) wiederum kommt in Pflanzen vorwiegend als biologisches Biocytin vor.

Chemisch definierte Folsäure (Vit. B9) erscheint im pflanzlichen Organismus in Form unterschiedlichster Folat-Moleküle, z.B. Pteroyldi-, -oligo- und -polyglutamate.

Vitamin B12 kommt in Pflanzenzellen generell nicht vor, liegt jedoch in Mikroorganismen z.B. als Methyl-, Cyano- und Adenosyl-Cobalamin vor.

Somit ist unter "organisch gebundenem Vitamin B" (oder organische Vitamin B Form - "Phyto-Vitamine" oder "Phytamine") erfindungsgemäß ein Vitamin B-Derivat, -Komplex oder -Variante zu verstehen, die sich zu synthetisch herstellbaren Vitaminen dadurch unterscheidet, dass in Pflanzen vorkommt - bzw. eine Mischung von Verbindungen darstellen, die unter die jeweilige Vitamin B Kategorie (B1, B2, etc.) fällt. "Organisch gebunden" ist nicht als "organisch-chemisch gebunden" zu verstehen, sondern bezieht sich auf die natürlich biologisch vorkommenden Formen der B Vitamine. Das erfindungsgemäße Verfahren kann zur Produktion von organisch gebundenem Vitamin B verwendet werden, die ggf. keine synthetisch herstellbaren Vitamine enthält, oder zur teilweisen oder vollständigen Umsetzung von synthetischen Vitaminen in ihre natürlichen biologischen Formen. Vorzugsweise wird das Verfahren bis zu einem Anteil von dem jeweiligen organisch gebundenen Vitamin B zum gesamten Vitamin B-Gehalt des jeweiligen Vitamins (z.B. Vitamin B1, B2, B3, B5, B6, B7, B9 oder B12) von mindestens 0,1%, 0,5%, 1%, 2%, 3%, 4%, 5%, 6%, 7%, 8%, 9%, 10%, 15%, 20%, 30% oder 40% durchgeführt oder Produkte mit diesen Anteilen zur Verfügung gestellt.

Während natürliche Vitamin-Komplexe für Vitamin C (z.B. aus Acerola-, Hibiskus- oder Hagebutten-Früchten), für Vitamin E (z.B. aus Soja oder Reiskeimen) sowie für Carotinoide (z.B. aus Karotten, Algen oder Palmöl) bereits bekannt sind, gibt es derzeit weltweit keine nennenswerte natürliche Quelle für die Vitamine der B-Gruppe, von Hefe- oder Leber-Extrakten abgesehen.

Somit wird gemäß der Erfindung bevorzugt eine Vitamin B-Variante ausgewählt aus Thiaminpyrophosphat, Thiamintriphosphat, FAD (Flavin adenin dinukleotid), FADH, FADH2 (reduzierte Formen von FAD), FMN (Flavin mononukleotid), NADH, NADPH, NAD oder NADP, Coenzym A, Pyridoxylphosphat und Biocytin, Pteroyldi-, -oligo- und -polyglutamate zur Verfügung gestellt, bzw. das Verfahren zur Anreicherung von Pflanzen mit diesen Vitaminen.

Bevorzugt werden die Pflanzensamen in der Lösung des jeweiligen Vitamins gekeimt. Bevorzugt werden die Pflanzensamen in der Lösung des jeweiligen Vitamins durch Einweichen vorgekeimt und anschließend durch regelmäßiges Besprühen der Keimung überlassen. Die Aufnahme von Vitamin B ist in den ersten Wachstumszyklen besonderes effizient.

Vorzugsweise werden die Pflanzensamen 1 Stunde bis 24 oder 48 Stunden, vorzugsweise maximal 10, 14, 16, 20, 24, 30, 36 oder 48 Stunden und mindestens 30 min, insbesondere 1, 2, 3 oder 4 Stunden, getränkt.

In speziellen Ausführungsformen ist das Masse-Verhältnis der Pflanzensamen zu der Lösung des jeweiligen Vitamins zwischen 1:1 bis 1:20, vorzugsweise bis 1:10, am meisten bevorzugt bis 1:5, ist. Insbesondere ist die Menge des Vitamins in der Lösung des jeweiligen Vitamins in Relation zu 100 g zu behandelnden Pflanzensamen größer als 0,01 mg, vorzugsweise größer als 0,1 mg, mehr bevorzugt größer als 1 mg, am meisten bevorzugt größer als 10 mg, und geringer als 20 g, vorzugsweise geringer als 5 g, am meisten bevorzugt geringer als 1 g.

In bevorzugten Ausführungsformen wird die Pflanze kultiviert und vorzugsweise mit einer Vitamin B-Lösung besprüht werden um die Vitamin Aufnahme zu vergrößern. Besprüht wird insbesondere über mehrere Tage, sodass die Keimung abgeschlossen wird, also der Samen verbraucht wird.

Die Pflanzen werden anschließend von anhaftendem Vitamin gereinigt um nicht in die organische Form umgesetztes Vitamin zu entfernen. In anderen Ausführungsformen werden die Pflanzensamen von der Keimung z.B. durch Trocknung abgehalten oder die Keimlinge unmittelbar nach der Keimung geerntet, z.B. nach dem die Pflanzen bis zu 0,5 cm, 1 cm, 2 cm oder 3 cm gewachsen sind - und insbesondere der Pflanzensamen aufgebraucht ist.

Bevorzugt wird anschließend das organisch gebundene Vitamin B isoliert. Dies kann auf herkömmlichem Wege wie Zerkleinerung der Pflanze, Extraktion und Umkristallisation erfolgen. Gegebenenfalls kann das Vitamin auch gereinigt werden, dies kann ebenfalls auf herkömmlichem Wege, wie z.B. Kristallisation oder Chromatographie, erfolgen.

Vorzugsweise ist der Pflanzensamen ausgewählt aus pflanzlichen, essbaren und keimfähigen Samenformen, insbesondere Gras-, Gemüse- und Getreidesamen.

Die keimfähigen Samen werden besonders bevorzugt ausgewählt aus Adzukibohnen, Amaranth, Luzerne (Alphalpha), Bitterkraut (Kresse), Bohnenarten, Brunnenkresse, Buchweizen, Erbsenarten, Boxhornklee, Soja, Gerste, Hafer, Hirse, Kürbis, Kichererbse, Kohlarten, Linsenarten, Leinsamenarten, Mais, Reis, Rettich, Roggen, Sesam, Senf, Sonnenblumen, Weizen und Quinoa.

Die mit Vitamin B angereicherten Pflanzenkeimlinge dienen als biologisch hochwertiger Rohstoff, vorzugsweise als Basis für Food-Supplements, diätetische Präparate, funktionelle Lebensmittel und Veterinärprodukte in fester, halbfester oder flüssiger Form, gegebenenfalls auch in magensaftresistenter Form.

Die vorliegende Erfindung wird durch die folgenden Beispiele näher erläutert.

### Beispiele:

Ziel der Untersuchungen war es, chemisch definierte Verbindungen von Vitaminen aus der B-Gruppe gezielt in pflanzliche Organismen einzubringen und einer Umwandlung in biologisch aktive Verbindungen zuzuführen. Als pflanzliche Anreicherungsmedien eigneten sich dazu insbesondere keimfähige, essbare Pflanzensamen. Essbare Keimlinge aus Getreide und anderen Pflanzensamen gelten nach aktuellen ernährungswissenschaftlichen Erkenntnissen als besonders wertvoll. Sie imponieren durch eine im Vergleich zu ungekeimten Samen bessere Protein-Qualität durch ein höherwertiges Gehaltsmuster an mehrfach ungesättigten Fettsäuren, vor allem aber durch eine bessere Bioverfügbarkeit der enthaltenen Vitamine, Mineralstoffe und Spurenelemente. Ziel der Versuche war es, keimbare Samen durch Anreicherung über standardisierte Nährlösungen gezielt mit Vitaminen des B-Komplexes anzureichern, um damit Keimlinge mit überdurchschnittlich hohen Gehalten an biologisch aktiven, zugleich aber auch standardisierten Gehalten an Vitaminen des B-Komplexes zu erhalten.

### Beispiel 1: Vorversuche

Zur Untersuchung der prinzipiellen Aufnahmefähigkeit verschiedener essbarer Keimlinge für B-Vitamine wurden keimbare Weizen-, Buchweizen- und Quinoa-Samen mit standardisierten wässrigen Lösungen von Niacin bzw. Cobalamin versetzt. Dabei wurde wie folgt vorgegangen:
Schritt 1: Jeweils 100 g Samen (Weizen, Buchweizen, Quinoa) wurden mit 2-fach destilliertem Wasser vorgewaschen.
Schritt 2: Jeweils 100 g der vorgewaschenen Samen-Art wurden in den jeweiligen Vitamin-Nährlösungen eingeweicht:
   - Weizen für 12 Stunden
   - Buchweizen für 16 Stunden
   - Quinoa für 1 Stunde
      Die Mengen der jeweiligen Einweich-Nährlösungen betrugen jeweils 500 ml.
Schritt 3: Nach der vorgegebenen Einweichzeit in den Vitamin-Nährlösungen wurden die vorgekeimten Samen über Filter abgeseiht und bei Raumtemperatur über 12 Stunden vorgekeimt.
Schritt 4: Jede einzelne Probe wurde 4 Tage lang jeweils 1 x täglich mit 25 ml frisch hergestellter Vitamin-Nährlösung besprüht.
Schritt 5: Nach 96 Stunden (4 Tagen) Keimdauer wurden die Keimlinge jeweils 3 x mit 800 ml 2-fach destilliertem Wasser gewaschen, um an der Oberfläche haftende Vitamin-Spuren damit restlos zu entfernen. Anschließend wurden die Proben bei 70 Grad Celsius 10 Stunden lang im Trockenschrank getrocknet.

Nachfolgende Tabelle 1 gibt die Vitaminkonzentrationen der Niacin- und Cobalamin- Nährlösungen an:

**Tabelle 1:**

| **Standards** | **Konz.1 [g/L]** | **Konz.2 [g/L]** | **Konz.3 [g/L]** | **Konz.4 [g/L]** | **Konz.5 [g/L]** | **Konz.6 [g/L]** |
|---|---|---|---|---|---|---|
| Niacin | 0,1 | 0,5 | 1 | 2 | 5 | 10 |
| Cobalamin | 0,001 | 0,005 | 0,01 | 0,02 | 0,05 | 0,1 |

### Beispiel 2: graduelle Steigerung der Vitamine

Die nachfolgende Tabelle 2 gibt die Niacin-Gehalte in mg/100g getrocknetem Keimling an.

W(0), B(0), Q(0) stehen für ungekeimten Weizen, Buchweizen und Quinoa. W(zdw), B(zdw), Q(zdw) stehen für die jeweiligen Keimlingsarten, die nach dem gleichen Verfahren wie die Prüflings-Keimlinge gekeimt wurden, allerdings nicht in vitaminhaltigen Nährlösungen, sondern in 2fach destilliertem Wasser.

Die Bezeichnungen (Konz.1) bis (Konz.6) symbolisieren die jeweiligen Niacin-Konzentrationen der Nährlösungen, in denen die jeweiligen Samen gekeimt wurden.

**Tabelle 2:**

| ***Proben*** | ***B3 [mg*/*100g Keimling]*** | ***Proben*** | ***B3 [mg*/*100g Keimling]*** | ***Proben*** | ***B3 [mg*/*100g Keimling]*** |
|---|---|---|---|---|---|
| W(0) | 4,1 | B(0) | 5,2 | Q(0) | 1,6 |
| W(zdw) | 5,4 | B(zdw) | 6,2 | Q(zdw) | 3,1 |
| W(Konz.1) | 7,9 | B(Konz.1) | 8 | Q(Konz.1) | 3,9 |
| W(Konz.2) | 17,5 | B(Konz.2) | 35,5 | Q(Konz.2) | 19,5 |
| W(Konz.3) | 74 | B(Konz.3) | 70,3 | Q(Konz.3) | 35,5 |
| W(Konz.4) | 195 | B(Konz.4) | 186 | Q(Konz.4) | 175 |
| W(Konz.5) | 400 | B(Konz.5) | 791 | Q(Konz.5) | 626 |
| W(Konz.6) | 729 | B(Konz.6) | 1680 | Q(Konz.6) | 1240 |

Tabelle 3 gibt - in analoger Symbolik - die Gehalts- bzw. Anreicherungs-Werte für Vitamin B12 an:

**Tabelle 3:**

| ***Proben*** | ***B12 [mg*/*100g Keimling]*** | ***Proben*** | ***B12 [mg*/*100g Keimling]*** | ***Proben*** | ***B12 [mg*/*100g Keimling]*** |
|---|---|---|---|---|---|
| W(0) | 4,8 8 | B(0) | 0,9 | Q(0) | 1,4 |
| W(zdw) | 74,7 | B(zdw) | 12,3 | Q(zdw) | 23,8 |
| W(Konz.1) | 90 | B(Konz.1) | 111 | Q(Konz.1) | 69 |
| W(Konz.2) | 349 | B(Konz.2) | 481 | Q(Konz.2) | 378 |
| W(Konz.3) | 658 | B(Konz.3) | 895 | Q(Konz.3) | 676 |
| W(Konz.4) | 580 | B(Konz.4) | 700 | Q(Konz.4) | 570 |
| W(Konz.5) | 3240 | B(Konz.5) | 1460 | Q(Konz.5) | 1580 |
| W(Konz.6) | 4170 | B(Konz.6) | 3010 | Q(Konz.6) | 2360 |

Die Erfassung der einzelnen Vitamin-Gehalte wurde vom Institut Kuhlmann, Hedwig-Laudien-Ring 3, D-67061 Ludwigshafen nach folgenden Analysenverfahren durchgeführt:
- Vitamin B1 mikrobiologisch mit Hanseniaspora uvarum
- Vitamin B2 mikrobiologisch mit Lactobacillus rhamnosus (AOAC 940.33)
- Vitamin B6 mikrobiologisch mit Neurospora sitophila
- Vitamin B12 mikrobiologisch mit Lactobacillus delbrückii (AOAC 952.20)
- Niacin mikrobiologisch mit Lactobacillus plantarum (AOAC 944.13) .
- Folsäure mikrobiologisch mit Enteroroccus hirae (AOAC 944.12)
- Pantothensäure mikrobiologisch mit Lactobacillus plantarum (AOAC 945.74)
- Biotin mikrobiologisch mit Neurospora crassa

### Beispiel 3: komplexe Vitamin Zusammensetzungen

Nachdem die Vorversuche mit den oben angeführten 3 Samenarten positiv verlaufen waren, wurden Quinoa-Samen nach einem analogen Verfahren mit einer komplexen Vitamin B-Nährlösung folgender Zusammensetzung (Tabelle 4) zur Keimung gebracht:
Vitamin-Gehalt in 1 Liter wässriger Nährlösung:

| Vitamine | mg/Liter |
|---|---|
| Vitamin B1 (Thiamin) | 1.500 |
| Vitamin B2 (Riboflavin) | 10.000 |
| Vitamin B3 (Niacin) | 22.000 |
| Vitamin B5 (Pantothensäure) | 25.000 |
| Vitamin B6 (Pyridoxin) | 3.300 |
| Vitamin B7 (Biotin) | 250 |
| Vitamin B9 (Folsäure) | 1.000 |
| Vitamin B12, Cyanocobalamin | 5 |

Die durch nach dem oben dargestellten Verfahren durchgeführte Waschung, Keimung und Trocknung erhaltenen Quinoa-Keimlinge ergaben nachfolgende Vitamin-Gehalte:

| Vitamine | mg/100g |
|---|---|
| Vitamin B1 (Thiamin) | 83,4 |
| Vitamin B2 (Riboflavin) | 134,0 |
| Vitamin B3 (Niacin) | 1300,0 |
| Vitamin B5 (Pantothensäure) | 793,0 |
| Vitamin B6 (Pyridoxin) | 155,0 |
| Vitamin B7 (Biotin) | 14,9 |
| Vitamin B9 (Folsäure) | 12,4 |
| Vitamin B12, Cyanocobalamin | 0,21 |

### Beispiel 4: Überführung der Vitamine

In einem weiteren Verfahrensschritt wurde untersucht, ob - in Abhängigkeit von der Keimdauer - ein Teil der während des Einweichens absorbierten Vitamine aus der Einweich-Tränklösung während des Keimungsprozesses in organisch gebundene Formen übergeführt wird. Dazu wurde 200 g keimfähige Quinoasamen für 6 Stunden in 320 ml einer Nährlösung folgender Zusammensetzung getränkt:
Vitamine gelöst in 1 l Wasser:

| Vitamine | mg/l Wasser |
|---|---|
| Thiaminhydrochlorid | 1.995 |
| Riboflavin | 10.000 |
| Niacinamid | 22.000 |
| Calcium D-Pantothenat | 27.747 |
| Pyridoxolhydrochlorid | 3.993 |
| Biotin | 250 |
| Folsäure | 1.090 |
| Cyanocobalamin | 5 |

Dabei wurden nach 30-stündiger Keimdauer und anschließendem gründlichen Waschen nach der mikrobiologischen VitaFast®-Methode der R-Biopharm GmbH / Institut für Produktqualität, Teltowkanalstraße 2, D-12047 Berlin, folgende Vitamin-Werte analysiert:

| ***Proben-nummer ifp*** | ***Codierung*** | | | ***Gesamt*** | ***Frei*** | ***Gebunden*** |
|---|---|---|---|---|---|---|
| 07/2138 | L 259 M | Niacin | (mg/100 g) | 881 | 865 | 16 |
| | | Vitamin B1 (ber. als Thiamin) | (mg/100 g) | 44 | 44 | 0 |
| | | Vitamin B2 (Riboflavin) | (mg/100 g) | 195 | 174 | 21 |

Nach 45-stündiger Keimdauer und anschließendem gründlichen Waschen zeigte sich - über die gleiche Analysenmethode - ein deutlicher Anstieg der Anteile an organisch gebundenen Vitaminen:

| ***Proben-nummer ifp*** | ***Codierung*** | | | ***Gesamt*** | ***Frei*** | ***Gebunden*** |
|---|---|---|---|---|---|---|
| 07/2139 | L 260 M | Niacin | (mg/100 g) | 959 | 910 | 49 |
| | | Vitamin B1 (ber. als Thiamin) | (mg/100 g) | 52 | 50 | 2 |
| | | Vitamin B2 (Riboflavin) | (mg/100 g) | 224 | 185 | 39 |

Das Vitafast®-Analyseverfahren wurde nach Lindeke (HYGIENE Report 2 (2006):4-6) durchgeführt.

## Patentansprüche

1. Verfahren zur Produktion von organisch gebundenem Vitamin B, vorzugsweise ausgewählt aus Vitamin B1, B2, B3, B5, B6, B7, B9, B12 oder Mischungen hiervon, in Pflanzen, wobei keimfähige Pflanzensamen in einer Lösung eines B Vitamins anfangs getränkt und während des Keimungsprozesses mit Vitamin B-Lösungen besprüht werden.

2. Verfahren nach Anspruch 1, **dadurch gekennzeichnet, dass** das organisch gebundene Vitamin eine Variante von Vitamin B1 umfasst, insbesondere Thiaminpyrophosphat oder Thiamintriphosphat.

3. Verfahren nach Anspruch 1 oder 2, **dadurch gekennzeichnet, dass** das organisch gebundene Vitamin eine Variante von Vitamin B2 umfasst, insbesondere FAD, FADH, FADH2 oder FMN.

4. Verfahren nach Anspruch 1 bis 3, **dadurch gekennzeichnet, dass** das organisch gebundene Vitamin eine Variante von Vitamin B3 umfasst, insbesondere NADH, NADPH, NAD oder NADP.

5. Verfahren nach Anspruch 1 bis 4, **dadurch gekennzeichnet, dass** das organisch gebundene Vitamin eine Variante von Vitamin B5 umfasst, insbesondere Coenzym A.

6. Verfahren nach Anspruch 1 bis 5, **dadurch gekennzeichnet, dass** das organisch gebundene Vitamin eine Variante von Vitamin B6 umfasst, insbesondere Pyridoxylphosphat.

7. Verfahren nach Anspruch 1 bis 6, **dadurch gekennzeichnet, dass** das organisch gebundene Vitamin eine Variante von Vitamin B7 umfasst, insbesondere Biocytin.

8. Verfahren nach Anspruch 1 bis 7, **dadurch gekennzeichnet, dass** das organisch gebundene Vitamin eine Variante von Vitamin B9 umfasst, insbesondere Pteroyldi-, -oligo- und -polyglutamate.

9. Verfahren nach Anspruch 1 bis 8, **dadurch gekennzeichnet, dass** das organisch gebundene Vitamin eine Variante von Vitamin B12 umfasst.

10. Verfahren nach einem der Ansprüche 1 bis 9, **dadurch gekennzeichnet, dass** die Pflanzensamen in der Lösung des jeweiligen Vitamins durch Anweichen angekeimt und durch nachträgliches Besprühen über mehrere Tage die Keimung abgeschlossen wird.

11. Verfahren nach einem der Ansprüche 1 bis 10, **dadurch gekennzeichnet, dass** die Pflanzensamen 1 Stunde bis 24 Stunden, vorzugsweise maximal 16 Stunden getränkt werden.

12. Verfahren nach einem der Ansprüche 1 bis 11, **dadurch gekennzeichnet, dass** das Masse-Verhältnis der Pflanzensamen zu der Tränklösung des jeweiligen Vitamins zwischen 1:1 bis 1:20, vorzugsweise bis 1:10, am meisten bevorzugt bis 1:5, ist.

13. Verfahren nach einem der Ansprüche 1 bis 12, **dadurch gekennzeichnet, dass** die Menge des Vitamins in der Lösung des jeweiligen Vitamins in Relation zu 100 g zu behandelnden Pflanzensamen größer als 0,01 mg, vorzugsweise größer als 0,1 mg, mehr bevorzugt größer als 1 mg, am meisten bevorzugt größer als 10 mg, und geringer als 20 g, vorzugsweise geringer als 5 g, am meisten bevorzugt geringer als 1 g, ist.

14. Verfahren nach einem der Ansprüche 1 bis 13, **dadurch gekennzeichnet, dass** die Pflanzen mehrtägig besprüht wird.

15. Verfahren nach einem der Ansprüche 1 bis 14, umfassend den Schritt der Isolierung von organisch gebundenem Vitamin B aus der Pflanze nach dem Tränken und Besprühen.

16. Verfahren nach einem der Ansprüche 1 bis 15, **dadurch gekennzeichnet, dass** der Pflanzensamen ausgewählt ist aus essbaren und keimfähigen Samenformen, insbesondere Gras-, Gemüse- und Getreidesamen.

17. Verfahren nach einem der Ansprüche 1 bis 16, **dadurch gekennzeichnet, dass** die Pflanze ausgewählt ist aus Adzukibohnen, Amaranth, Luzerne, Kresse, Bohnenarten, Brunnenkresse, Buchweizen, Erbsenarten, Boxhornklee, Soja, Gerste, Hafer, Hirse, Kürbis, Kichererbse, Kohlarten, Linsenarten, Leinsamenarten, Mais, Reis, Rettich, Roggen, Sesam, Senf, Sonnenblumen, Weizen und Quinoa.

18. Verfahren nach einem der Ansprüche 1 bis 17, **dadurch gekennzeichnet, dass** die Pflanzenkeimlinge nach dem Tränken und Besprühen zu.einem biologischen Rohstoff, vorzugsweise als Basis für Food-Supplements, diätetische Präparate, funktionelle Lebensmittel und Veterinärprodukte, in fester, halbfester oder flüssiger Form, gegebenenfalls auch in magensaftresistenter Form, verarbeitet werden.

19. Verwendung eines Verfahrens nach einem der Ansprüche 1 bis 18 zur Produktion von organisch gebundenem Vitamin B.

## Claims

1. A method for producing organically bound vitamin B, preferably selected from vitamin B1, B2, B3, B5, B6, B7, B9, B12, or mixtures thereof, in plants, wherein germinable plant seeds are at first soaked in a solution of a vitamin B and are sprinkled with vitamin B solutions during the germination process.

2. The method according to claim 1, **characterised in that** the organically-bound vitamin comprises a variant of vitamin B1, in particular thiamine pyrophosphate or thiamine triphosphate.

3. The method according to claim 1 or 2, **characterised in that** the organically bound vitamin comprises a variant of vitamin B2, in particular FAD, FADH, FADH2 or FMN.

4. The method according to claims 1 to 3, **characterised in that** the organically bound vitamin comprises a variant of vitamin B3, in particular NADH, NADPH, NAD or NADP.

5. The method according to claims 1 to 4, **characterised in that** the organically bound vitamin comprises a variant of vitamin B5, in particular co-enzyme A.

6. The method according to claims 1 to 5, **characterised in that** the organically bound vitamin comprises a variant of vitamin B6, in particular pyridoxyl phosphate.

7. The method according to claims 1 to 6, **characterised in that** the organically bound vitamin comprises a variant of vitamin B7, in particular biocytin.

8. The method according to claims 1 to 7, **characterised in that** the organically bound vitamin comprises a variant of vitamin B9, in particular pteroyldiglutamate, pteroyloligoglutamate and pteroylpolyglutamate.

9. The method according to claims 1 to 8, **characterised in that** the organically bound vitamin comprises a variant of vitamin

10. The method according to any one of claims 1 to 9, **characterised in that** the plant seeds are caused to begin to germinate by being pre steeped in the solution of the respective vitamin, and the germination process is completed by subsequent sprinkling over several days.

11. The method according to any one of claims 1 to 10, **characterised in that** the plant seeds are soaked for 1 hour to 24 hours, preferably 16 hours at the most.

12. The method according to any one of claims 1 to 11, **characterised in that** the mass ratio of the plant seeds to the soak solution of the respective vitamin is from 1:1 to 1:20, preferably up to 1:10, most preferred up to 1:5.

13. The method according to any one of claims 1 to 12, **characterised in that**, in relation to 100 g of the plant seeds to be treated, the portion of the vitamin in the solution of the respective vitamin is greater than 0.01 mg, preferably greater than 0.1 mg, more preferred greater than 1 mg, most preferred greater than 10 mg, and less than 20 g, preferably less than 5 g, most preferred less than 1 g.

14. The method according to any one of claims 1 to 13, **characterised in that** the plants are sprinkled over several days.

15. The method according to any one of claims 1 to 14, comprising the step of isolating organically bound vitamin B from the plant after soaking and sprinkling.

16. The method according to any one of claims 1 to 15, **characterised in that** the plant seed is selected from edible and germinable kinds of seeds, in particular grass, vegetable and cereal seeds.

17. The method according to any one of claims 1 to 16, **characterised in that** the plant is selected from azuki bean, amaranth, lucerne, cress, varieties of beans, watercress, buckwheat, varieties of peas, fenugreek, soya, barley, oat, millet, pumpkin, chickpea, varieties of cabbage, varieties of lentils, varieties of linseeds, corn, rice, radish, rye, sesame, mustard, sun flowers, wheat and quinoa.

18. The method according to any one of claims 1 to 17, **characterised in that**, after soaking and sprinkling, the seedlings are processed to a biologically high-quality raw material, preferably as basis for food supplements, dietary preparations, functional food and veterinary products, in solid, semi-solid or liquid form, optionally also in gastric-juice-resistant form.

19. The use of a method according to any one of claims 1 to 18 for producing organically bound vitamin B.

## Revendications

1. Procédé de production de vitamine B organique sous forme liée, choisie de préférence parmi les vitamines B1, B2, B3, B5, B6, B7, B9, B12 ou des mélanges de celles-ci, dans des plantes, dans lequel on trempe d'abord des graines de plantes aptes à germer dans une solution d'une vitamine B et pendant le processus de germination on pulvérise sur celles-ci des solutions de vitamine B.

2. Procédé selon la revendication 1, **caractérisé en ce que** la vitamine organique sous forme liée comprend une variante de la vitamine B1, en particulier le pyrophosphate de thiamine ou le triphosphate de thiamine.

3. Procédé selon la revendication 1 ou 2, **caractérisé en ce que** la vitamine organique sous forme liée comprend une variante de la vitamine B2, en particulier FAD, FADH, FADH2 ou FMN.

4. Procédé selon l'une quelconque des revendications 1 à 3, **caractérisé en ce que** la vitamine organique sous forme liée comprend une variante de la vitamine B3, en particulier NADH, NADPH, NAD ou NADP.

5. Procédé selon l'une quelconque des revendications 1 à 4, **caractérisé en ce que** la vitamine organique sous forme liée comprend une variante de la vitamine B5, en particulier la coenzyme A.

6. Procédé selon l'une quelconque des revendications 1 à 5, **caractérisé en ce que** la vitamine organique sous forme liée comprend une variante de la vitamine B6, en particulier le phosphate de pyridoxyle.

7. Procédé selon l'une quelconque des revendications 1 à 6, **caractérisé en ce que** la vitamine organique sous forme liée' comprend une variante de la vitamine B7, en particulier la biocytine.

8. Procédé selon l'une quelconque des revendications 1 à 7, **caractérisé en ce que** la vitamine organique sous forme liée comprend une variante de la vitamine B9, en particulier le di-, l'oligo- et le polyglutamate de ptéroyle.

9. Procédé selon l'une quelconque des revendications 1 à 8, **caractérisé en ce que** la vitamine organique sous forme liée comprend une variante de la vitamine B12.

10. Procédé selon l'une quelconque des revendications 1 à 9, **caractérisé en ce que** les graines de plantes commencent à germer en se ramollissant dans la solution de la vitamine respective et la germination est achevée par pulvérisation ultérieure pendant plusieurs jours.

11. Procédé selon l'une quelconque des revendications 1 à 10, **caractérisé en ce que** les graines de plantes sont trempées pendant 1 heure à 24 heures, de préférence au maximum 16 heures.

12. Procédé selon l'une quelconque des revendications 1 à 11, **caractérisé en ce que** le rapport massique des graines de plantes à la solution de trempage de la vitamine respective est compris entre 1:1 et 1:20, de préférence jusqu'à 1:10, de façon particulièrement préférée jusqu'à 1:5.

13. Procédé selon l'une quelconque des revendications 1 à 12, **caractérisé en ce que** la quantité de vitamine dans la solution de la vitamine respective par rapport à 100 g de graines de plantes à traiter est supérieure à 0,01 mg, de préférence supérieure à 0,1 mg, encore mieux supérieure à 1 mg, de façon particulièrement préférée, supérieure à 10 mg, et inférieure à 20 g, de préférence inférieure à 5 g, de façon particulièrement préférée, inférieure à 1 g.

14. Procédé selon l'une quelconque des revendications 1 à 13, **caractérisé en ce que** les plantes reçoivent une pulvérisation pendant plusieurs jours.

15. Procédé selon l'une quelconque des revendications 1 à 14, comprenant l'étape de l'isolement de vitamine B organique sous forme liée à partir de la plante, après le trempage et la pulvérisation.

16. Procédé selon l'une quelconque des revendications 1 à 15, **caractérisé en ce que** la graine de plante est choisie parmi des types de graines aptes à germer et comestibles, en particulier des graines de graminées, de légumes et de céréales.

17. Procédé selon l'une quelconque des revendications 1 à 16, **caractérisé en ce que** la plante est choisie parmi le haricot adzuki, l'amarante, la luzerne, le cresson, les variétés de haricot, le cresson de fontaine, le sarrasin, les variétés de pois, le lyciet de Barbarie, le soja, l'orge, l'avoine, le millet, la courge, le poids chiche, les variétés de chou, les variétés de lentille, les variétés de lin, le maïs, le riz, le radis, le seigle, le sésame, la moutarde, le tournesol, le blé et la quinoa.

18. Procédé selon l'une quelconque des revendications 1 à 17, **caractérisé en ce que** le plantules sont transformées, après le trempage et la pulvérisation, en une matière première biologique, de préférence en tant que base de compléments alimentaires, de préparations diététiques, de produits alimentaires fonctionnels et de produits vétérinaires, sous forme solide, semi-solide ou liquide, éventuellement également sous forme résistante au suc gastrique.

19. Utilisation d'un procédé selon l'une quelconque des revendications 1 à 18, pour la production de vitamine B organique sous forme liée.
